# EUROPÄISCHE PATENTANMELDUNG

(11) **EP 3 441 000 A1**
(43) Veröffentlichungstag der Anmeldung: **13.02.2019**
(21) Anmeldenummer: 17207751.3
(22) Anmeldetag: 15.12.2017
(51) Int. Cl.: A61B 5/055, A61B 5/00, G01R 33/28, G06Q 20/18

(54) **VORRICHTUNG ZUR ERFASSUNG, BEARBEITUNG UND PRÄSENTATION VON DETAILINFORMATIONEN ÜBER DEN KÖRPERZUSTAND**

(30) Priorität: 08.08.2017 EP 17185317
(71) Anmelder: Davis, Edwin, 40667 Meerbusch (DE)
(72) Erfinder: Davis, Edwin, 40667 Meerbusch (DE)
(74) Vertreter: Rausch Wanischeck-Bergmann Brinkmann

(57) **Zusammenfassung**

Die Erfindung betrifft eine Vorrichtung zur Erfassung, Bearbeitung und Präsentation von Detailinformationen über den Körperzustand eines Benutzers, umfassend ein Gehäuse, in welchem wenigstens eine Rechnereinheit, eine Aktivierungseinheit, ein Display sowie eine Sensorschnittstelle angeordnet sind, und wenigstens einen Sensor, dadurch gekennzeichnet, dass der Sensor ein Magnetresonanz-Sensor ist.

## Beschreibung

Die vorliegende Erfindung betrifft eine Vorrichtung zur Erfassung, Bearbeitung und Präsentation von Detailinformationen über den Körperzustand eines Benutzers, umfassend ein Gehäuse, in welchem eine Rechnereinheit, eine Aktivierungseinheit, ein Display sowie eine Sensorschnittstelle angeordnet sind. Darüber hinaus ist über die Sensorschnittstelle wenigstens ein Sensor mit der Vorrichtung verbunden.

Derartige Vorrichtungen sind im Stand der Technik bekannt. Beispielsweise ein mit einem Blutdruckmessgerät versehener Rechner kann über die Tastatur aktiviert werden und kann die Messwerte auf den Monitor anzeigen. Allerdings liegt hier keine Gehäuseeinheit vor. So können eine Vielzahl von Körperfunktionen, beispielsweise Blutdruck, Herzfrequenz, Körpertemperatur und so weiter mit unterschiedlichen Geräten ermittelt werden. Allgemein werden Informationen über den Gesundheitszustand als Bioinformationen bezeichnet. Eine Vielzahl unterschiedlichster Geräte dienen der Erfassung von Bioinformationen und Informationen über Körperfunktionen. Die Geräte sind teils mobil, teils stationär.

Blutanalysen und dergleichen werden in Labors und von Fachpersonal vorgenommen. Die wesentlichen Analysen von Bioinformationen, Körperfunktionen und dergleichen werden durch Ärzte unter Zuhilfenahme unterschiedlichster Geräte ermittelt und interpretiert.

In jüngerer Zeit gibt es eine Vielzahl von Geräten, um beispielsweise eine Fitnesszustand zu ermitteln, zu überwachen und den Benutzer zu entsprechendem Bewegungsverhalten anzuhalten. Eine Vielzahl derartiger rechnergestützter Informationen stehen der Person zur Verfügung. Eine Vielzahl solcher Vorgänge können internetbasierend abgewickelt werden.

Gleichwohl besteht nach wie vor ein Bedarf nach einem möglichst umfassend Zustandsbericht außerhalb der medizinischen Überwachung einer Person. Dies betrifft zum einen das gesteigerte Diätbewusstsein, ein gesteigertes Bewegungsprofil und darüber hinaus eine umfassende Verfügbarkeit von Ernährungszusätzen. In den seltensten Fällen haben die Benutzer, also Menschen, Erkenntnisse über den Zusammenhang ihrer Ernährung, ihres Bewegungsverhaltens und ihres mentalen Verhaltens zum Körperbefinden und sich ergebende Körperzustandsinformationen.

Das bedeutet, dass eine Vielzahl von Menschen Nahrungszusätze zu sich nehmen, beispielsweise Superfood, Heilpflanzenextrakte und dergleichen, ohne wirklich erfassen zu können, wie diese wirken. Dies würde einen ständigen Überwachungsvorgang durch einen Mediziner erfordern.

So ist es ohne Weiteres denkbar, dass eine Vielzahl von Nahrungszusätzen Nebenwirkungen haben, die andere Aspekte beeinflussen. So können beispielsweise Schwermetalle abführende oder abbauende Pflanzen unbeabsichtigt auch andere Produkte abbauen, so dass der Benutzer beispielsweise bestimmte Vitaminmängel herbeiführt oder dergleichen.

Es besteht insgesamt ein Bedarf an Möglichkeiten, den aktuellen Körperzustand genauer zu überwachen.

Davon ausgehend, dass es wenig sinnvoll ist, den Benutzern eine große Zahl von möglichen Überwachungsgeräten an die Hand zu geben und diesen mit der Interpretation im Wesentlichen allein zu lassen, liegt der vorliegenden Erfindung die **Aufgabe** zugrunde, eine Vorrichtung der gattungsgemäßen Art dahingehend zu verbessern, dass diese zum einen mit geringem Aufwand und in kurzer Zeit Detailinformationen über den Körperzustand eines Benutzers erfassen und präsentieren kann, darüber hinaus aber geeignet ist, eine standardisierte zentrale Auswertung einer großen Vielzahl von Detailinformationen vornehmen zu können, um geeignete richtungsweisende Verhaltensempfehlungen abgeben zu können.

Zur technischen **Lösung** dieser Aufgabe wird eine Vorrichtung mit den Merkmalen des Patentanspruches 1 vorgeschlagen. Weitere Vorteile und Merkmale ergeben sich auch den Unteransprüchen.

Erfindungsgemäß wird eine Gehäuseeinheit vorgesehen, welche wenigstens einen nutzbaren Sensor aufweist. Dieser ist über eine Sensorschnittstelle mit einer im Gehäuse angeordneten Rechnereinheit verbunden. Eine Aktivierungseinheit dient dem Zweck, einen Testlauf zu initialisieren. Die Aktivierungseinheit kann ein einzelnes Element umfassen, beispielsweise im einfachsten Fall eine Tastatur, einen Startknopf oder dergleichen, oder auch alternativ oder ergänzend andere Einheiten wie Kartenleser, Scanner oder dergleichen.

Erfindungsgemäß ist wenigstens einer der Sensoren ein Magnetresonanzsensor.

Magnetresonanzsensoren sind in der Vergangenheit als aufwendige medizinische Apparate im Einsatz, können aber inzwischen auch als durch jedermann anwendbare Funktionseinheit ausgestaltet sein.

Dem Verfahren der Magnetresonanz liegt zugrunde, dass der menschliche Körper ununterbrochen elektromagnetische Signale aussendet. Der menschliche Körper umfasst eine große Anzahl von Zellen, welche in einem ständige Veränderungsprozess befindlich sind. Sie wachsen, entwickeln sich, teilen sich, trennen sich, regenerieren sich und vermehren sich durch Teilung ständig selbst. Man geht davon aus, dass in einem menschlichen Körper in einer Sekunde in etwa 25 Millionen Zellen solchen Prozessen unterworfen sind. Blutzellen erneuern sich in einer Rate von zirka 100 Mio. pro Minute. Auf atomarer Ebene sind Atomkerne und Elektronen umfasst, welche sich in ständiger Hochgeschwindigkeitsbewegung und Veränderung befinden und dabei beständig elektromagnetische Wellen aussenden.

Die elektromagnetischen Wellen lassen sich differenzieren und unterscheiden sich je nach Körperzustand. Die zunehmende Bestimmbarkeit der elektrischen Wellensignale erlaubt einen beständig verbesserten Rückschluss auf den Körperstatus des Benutzers. Auf biomolekularer Ebene geht man im Bereich der Quantenmedizin davon aus, dass alle Elektronen geladene Körper sind und der Spinn des Elektrons und Veränderungen im Orbit Veränderungen der vom Atom imitierten elektromagnetischen Strahlung bedeuten. Es handelt sich um eine Veränderung der Energie der elektromagnetischen Strahlung, welche erfassbar ist.

Durch empirische Ermittlungen hat man inzwischen herausgefunden, dass unterschiedliche Bereiche des Körpers auf unterschiedliche Frequenzen ansprechen. Man sendet also Signale aus und überwacht die Reflektionen. Auf diese Weise ist es nach Verstärkung der Signale möglich, durch Resonanzanalyse Rückschlüsse auf konkrete Krankheiten, den Zustand bestimmter Körperbereiche und dergleichen rückzuschließen.

Erfindungsgemäß ist ein solcher Sensor Bestandteil der Vorrichtung. Die Vorrichtung kann als Steele ausgebildet sein, wobei die Steele die Gehäuseeinheit ist, die die wenigstens eine Rechnereinheit, die Aktivierungseinheit, die Sensorschnittstelle und das Display umfasst. Das Display ist vorzugsweise in einer mittleren Augenhöhe angeordnet, so dass eine große Vielzahl von Benutzern das Display ohne Weiteres sehen können. Das Display kann vertikal verschiebbar und/oder schwenkbar angeordnet sein.

Erfindungsgemäß ist eine Tastatureinheit angeordnet, welche beispielsweise in einen horizontalen Flächenbereich des Gehäuses beziehungsweise der Steele angeordnet sein kann. Auch dieser Bereich kann verstellbar ausgebildet sein.

In vorteilhafter Weise kann die Vorrichtung ein Kartenlesegerät oder einen Scanner aufweisen.

Weiterhin wird mit Vorteil vorgeschlagen, dass die Rechnereinheit netzwerkfähig ausgerüstet ist. Darunter ist im Sinne der vorliegenden Anmeldung zu verstehen, dass die Einheit sowohl WLAN-fähig als auch internetfähig ausgebildet ist beziehungsweise zur drahtlosen Datenkommunikation beispielsweise über ein Mobilfunknetz geeignet ist. Auf diese Weise kann die Vorrichtung sozusagen online überwacht oder im Datendialog mit einem Zentralrechner verbesserte Analysen, Präsentationen oder Empfehlungen bereitstellen.

Die Rechnereinheit weist vorzugsweise eine Software zur Bearbeitung der Sensordaten und zur Präsentation auf dem Display auf. Die Rechnereinheit ist selbstverständlich geeignet, den Sensor entsprechend zu betreiben, so dass dieser die gewünschten Signale aussenden und empfangen kann.

In vorteilhafter Weise kann eine zweite Rechnereinheit zur Weiterverarbeitung der von der ersten Rechnereinheit gelieferten Daten vorgesehen sein. In diesem Fall ist die erste Rechnereinheit eine Steuereinheit zur Steuerung des Sensors und zum Abgreifen seiner Daten. Diese können vorverarbeitet und dann an einer Schnittstelle an eine zweite Rechnereinheit zum Zwecke der Weiterbearbeitung und Präsentation übergeben werden.

In vorteilhafter Weise umfasst die Präsentation sowohl Textinformationen als auch Zustandssignale. Die Zustandssignale können Ampelsignale umfassen, das heißt sie können durch farbliche Kennzeichnung beispielsweise rot-gelb-grün oder weitere Kombinationen auf besondere Ergebnisse und deren statistische Einordnung verweisen.

In vorteilhafter Weise werden die Sensordaten statistisch mit Datenbanken ausgewertet, welche eine große Zahl bisheriger Überprüfungen repräsentieren. Auf diese Weise kann ein Normalwert und die Abweichung der Messung vom Normalwert ins Positive oder ins Negative dargestellt werden.

Gemäß der Erfindung kann die Vorrichtung einen Touchscreen aufweisen. In vorteilhafter Weise kann die Steele über einen oder mehrere Monitore verfügen. So kann eine große Fläche in mehrere Teilbereiche unterteilt sein, die zum Teil der Darstellung von Werbung in Form von Videos, Bannern und dgl. dienen und einen Touchscreenbereich aufweisen. In dem Touchscreenbereich kann beispielsweise ein Nutzer seine Daten eingeben und den Ablauf starten. Weiterhin kann ein Bereich dem Zweck dienen, den Nutzer über die Bedienung zu informieren. Im Falle der Verwendung eines Touchscreens kann die Anordnung eines horizontalen Bereiches für eine Tastatur beispielsweise entfallen.

Gemäß einem weiteren vorteilhaften Vorschlag der Erfindung ist eine Halterung am Gehäuse angeordnet, in welcher ein Magnetresonanzsensor einsetzbar oder einlegbar ist. In vorteilhafter Weise kann dieser schwenkbar am Gehäuse angeordnet sein, um auf diese Weise ein sicheres Verstauen des Sensors zu ermöglichen.

Mit der Erfindung wird eine mit überschaubaren wirtschaftlichem Aufwand erstellbare Vorrichtung zur Erfassung, Bearbeitung und Präsentation von Detailinformationen über den Körperzustand eines Benutzers bereitgestellt, welche einem Benutzer eine Hilfestellung über sein Bewegungsverhalten, sein Ernährungsverhalten und dergleichen geben kann und ihm insbesondere ein Signal geben kann, wenn er einen bestimmten Bereich seiner Körperfunktionen medizinisch überprüfen lassen sollte.

In vorteilhafter Weise hat die erfindungsgemäße Vorrichtung eine Kamera und kann das Image des Benutzers erfassen. Die über den Sensor erfassten Informationen können in einer dem Benutzer zuzuordnenden Datenbank gespeichert werden. Hinzu können weitere Informationen erfasst werden, so dass eine zentrale Nutzerdatei entsteht. All diese Informationen können im Sinne einer zentralen Patientenakte auch diagnostische oder sonstige Bemerkungen bei Arztbesuchen, Fotos, Röntgenbilder und dergleichen enthalten, ebenso wie Messwerte, Berichte und so weiter. Eine derartige zentrale Aufbereitung und Speicherung von Informationen kann nutzerabhängig unter Berücksichtigung von Datenschutzbestimmungen gespeichert und gepflegt werden und nach Vorgaben des Nutzers bereitgestellt werden. Darüber hinaus können Logdateien mit Bezug auf den Verbrauch des Nutzers an Arzneimitteln, Nahrungsergänzungsmitteln und dergleichen ergänzt werden, die wiederum Schnittstellen zu Vertriebsplattformen eröffnen.

In erfindungsgemässer Weise ist die Vorrichtung zur NFC- Nutzung (near field communication) ausgerüstet. So können beispielsweise Handy-Zahlungsmethoden umgesetzt werden. Zu diesem Zweck weist sie wenigstens einen geeigneten Chip auf.

Die mittels des Sensors zu erfassenden Informationen können in den Bereichen Gesundheit, Fitness oder Schönheit (beauty) zugeordnet sein.

Insofern kann die erfindungsgemäße Vorrichtung Bestandteil eines Gesamtsystems sein, welches unter Nutzung von Zentralrechenanlagen und der entsprechenden Datenkommunikation, Wifi und dergleichen, nutzerbezogene Körper- und Gesundheitsinformationen speichert, pflegt, bereitstellt und ergänzt, wobei ein solches System offen sein kann für entsprechende Anbindungen an Gesundheitssysteme, Kassensysteme und dergleichen.

Im Sinne der Erfindung umfasst diese auch ein Verfahren zur Erfassung von Detailinformationen über den Körperzustand eines Benutzers mittels wenigstens eines Sensors, der in jedem Fall einen Magnetresonanzsensor aufweist. Im Rahmen dieses Verfahrens können die ermittelten Informationen gespeichert und benutzerbezogen aufbereitet und abgefragt werden beziehungsweise zur Verfügung gestellt werden. Diese Informationen können den gesamten Gesundheitsbereich des Benutzers umfassen und um diese anwachsen. Verfahrensgemäß kann auf diese Weise eine zentrale personenbezogene Akte erstellt, geführt und gepflegt werden. Im Rahmen des Verfahrens können die Informationen zum Zwecke der Ermittlung von Ergänzungsbedarf jeglicher Art, Vitamine, Heilmittel und dergleichen ausgewertet werden. Weitere Vorteile und Merkmale der Erfindung ergeben sich aus der folgenden Beschreibung anhand der Figuren. Dabei zeigen:

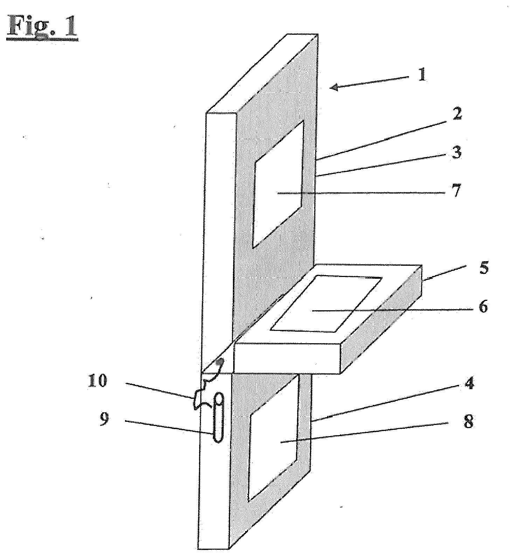

- Fig. 1: eine schematische Darstellung eines Ausführungsbeispiels für eine erfindungsgemäße Vorrichtung.

In Figur 1 ist schematisch eine Steele 1 gezeigt, welche aus einem Steelenkörper 2 besteht, der einen oberen Teil 3 und einen unteren Teil 4 umfasst. Im mittleren Bereich ist ein im Wesentlichen horizontal angeordneter Flächenbereich 5 gezeigt. Dieser kann am Steelenkörper 2 bewegbar oder verschwenkbar angeordnet sein, beispielsweise höhenverstellbar. Der Steelenkörper 2 seinerseits steht im Wesentlichen vertikal, kann aber je nach Aufstellungsort und Fixierungsart auch schräg angeordnet sein, an der Wand hängen oder dergleichen. Im oberen Bereich ist ein Display 7 angedeutet, welches der Präsentation von Daten und der Ablaufsteuerung durch Texthinweise und dergleichen dienen kann. Er kann auch dazu verwendet werden, Formulare auszufüllen, beispielsweise um den Benutzer zu identifizieren oder zu registrieren.

Auf dem horizontalen Bereich 5 ist eine Tastatur 6 angedeutet, mit welcher der Dialog geführt werden kann. Im unteren Bereich 4 ist eine Werbefläche 8 ausgebildet. Weitere Werbeflächen können auf der Rückseite, auf Seitenflächen und dergleichen angeordnet sein.

Ein Magnetresonanzsensor 9 in Form eines dünnen Stabes ist mittels einem Kabel 10 an der Steele befestigt. Auch kann eine Handauflage als Sensorfeld ausgebildet sein.

Ein nicht gezeigter Benutzer kann nun die Vorrichtung beispielsweise durch eine Tasteneingabe mittels der Tastatur 6 aktivieren. Er wird dann menügeführt, bis er den Sensor 9 in die Hand nimmt und abwartet, bis der Erfassungsvorgang abgeschlossen ist. Dann werden ihm beispielsweise auf dem Display 7 die Ergebnisse angezeigt. Auf Wunsch kann mittels eines nicht gezeigten Druckers, der ebenfalls im Bereich der Steele angeordnet sein kann, ein Ausdruck erfolgen. Auch ist eine Emailübermittlung auf den Emailaccount eines Benutzers möglich oder eine Kommunikation unter Nutzung einer App..

Die Ergänzung der Vorrichtung um weitere Sensoren, die nicht gezeigt sind, liegt im Bereich der Erfindung. So können beispielsweise Scanner für den Augenscan, Kameras, Temperatursensoren und so weiter eingesetzt sein.

Das beschriebene Ausführungsbeispiel dient nur der Erläuterung und ist nicht beschränkend.

### Bezugszeichen

- 1: Vorrichtung
- 2: Steele
- 3: oberer Bereich
- 4: unterer Bereich
- 5: mittlerer Bereich
- 6: Tastatur
- 7: Display
- 8: Werbefläche
- 9: Magnetresonanzsensor
- 10: Kabel

## Patentansprüche

1. Vorrichtung zur Erfassung, Bearbeitung und Präsentation von Detailinformationen über den Körperzustand eines Benutzers, umfassend ein Gehäuse, in welchem wenigstens eine Rechnereinheit, eine Aktivierungseinheit, ein Display sowie eine Sensorschnittstelle angeordnet sind, und wenigstens einen Sensor, **dadurch gekennzeichnet, dass** der Sensor ein Magnetresonanz-Sensor ist.

2. Vorrichtung nach Anspruch 1, **dadurch gekennzeichnet, dass** das Gehäuse eine Steele ist, in welcher die wenigstens eine Rechnereinheit, die Aktivierungseinheit, die wenigstens eine Sensorschnittstelle und in Augenhöhe das Display angeordnet sind.

3. Vorrichtung nach einem der vorhergehenden Ansprüche, **dadurch gekennzeichnet, dass** diese einen Touchscreen aufweist.

4. Vorrichtung nach einem der vorhergehenden Ansprüche, **dadurch gekennzeichnet, dass** diese eine Tastatur aufweist.

5. Vorrichtung nach einem der vorhergehenden Ansprüche, **dadurch gekennzeichnet, dass** diese ein Kartenlesegerät aufweist.

6. Vorrichtung nach einem der vorhergehenden Ansprüche, **dadurch gekennzeichnet, dass** der Magnetresonanz-Sensor stabförmig ausgebildet ist.

7. Vorrichtung nach einem der vorhergehenden Ansprüche, **dadurch gekennzeichnet, dass** diese eine Halterung für den Magnetresonanzsensor aufweist.

8. Vorrichtung nach Anspruch 7, **dadurch gekennzeichnet, dass** die Halterung schwenkbar am Gehäuse angeordnet ist.

9. Vorrichtung nach einem der vorhergehenden Ansprüche, **dadurch gekennzeichnet, dass** die Rechnereinheit netzwerkfähig ausgerüstet ist.

10. Vorrichtung nach einem der vorhergehenden Ansprüche, **dadurch gekennzeichnet, dass** die Steele zusätzliche Werbeflächen-Bereiche aufweist.

11. Vorrichtung nach einem der vorhergehenden Ansprüche, **dadurch gekennzeichnet, dass** die Rechnereinheit mit einer Software zur Bearbeitung der Sensordaten und zur Präsentation auf dem Display ausgestattet ist.

12. Vorrichtung nach einem der vorhergehenden Ansprüche, **dadurch gekennzeichnet, dass** diese eine Rechnereinheit zur Erfassung und Vor-Bearbeitung der Sensordaten und eine zweite Rechnereinheit zur Weiter-Bearbeitung der Sensordaten und deren Präsentation aufweist.

13. Vorrichtung nach einem der vorhergehenden Ansprüche, **dadurch gekennzeichnet, dass** die Präsentation Textinformationen und Zustandssignale umfasst.

14. Vorrichtung nach Anspruch 13, **dadurch gekennzeichnet, dass** die Zustandssignale Ampelsignale umfassen.

15. Vorrichtung nach einem der vorhergehenden Ansprüche, **dadurch gekennzeichnet, dass** der Magnetresonanzsensor als Handauflagefeld ausgebildet ist.
